(19)
Europäisches
Patentamt

European
Patent Office

Office européen
des brevets

(11) **EP 2 726 876 B1**

(12) ## EUROPEAN PATENT SPECIFICATION

(45) Date of publication and mention
of the grant of the patent:
**11.10.2017 Bulletin 2017/41**

(51) Int Cl.:
*G01N 33/543* (2006.01)      *G01N 21/55* (2014.01)
*G01N 21/77* (2006.01)      *G01N 21/552* (2014.01)

(21) Application number: **12805115.8**

(22) Date of filing: **27.06.2012**

(86) International application number:
**PCT/SE2012/050717**

(87) International publication number:
**WO 2013/002717 (03.01.2013 Gazette 2013/01)**

(54) **METHOD OF DETERMINING ACTIVE CONCENTRATION BY CALIBRATION-FREE ANALYSIS**

VERFAHREN ZUR ERMITTLUNG EINER AKTIVEN KONZENTRATION DURCH
KALIBRATIONSFREIE ANALYSE

PROCÉDÉ DE DÉTERMINATION D'UNE CONCENTRATION ACTIVE PAR ANALYSE SANS
ÉTALONNAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.06.2011  SE 1150612**

(43) Date of publication of application:
**07.05.2014  Bulletin 2014/19**

(73) Proprietor: **GE Healthcare Bio-Sciences AB
75184 Uppsala (SE)**

(72) Inventors:
• **KARLSSON, Robert
S-751 84 Uppsala (SE)**
• **ROOS, Håkan
S-751 84 Uppsala (SE)**

(74) Representative: **Brann AB
P.O. Box 3690
Drottninggatan 27
103 59 Stockholm (SE)**

(56) References cited:
**WO-A1-2004/109284      WO-A1-2009/025680
WO-A1-2009/025680      WO-A1-2011/049530
US-A1- 2005 131 650**

• **KRISTMUNDUR SIGMUNDSSON ET AL:
"Determination of Active Concentrations and
Association and Dissociation Rate Constants of
Interacting Biomolecules: An Analytical Solution
to the Theory for Kinetic and Mass Transport
Limitations in Biosensor Technology and Its
Experimental Verification", BIOCHEMISTRY,
AMERICAN CHEMICAL SOCIETY, US, vol. 41, 1
January 2002 (2002-01-01), pages 8263-8276,
XP008155383, ISSN: 0006-2960, DOI:
10.1021/BI020099H [retrieved on 2002-06-06]**
• **KARLSSON R ET AL: "Analyzing a kinetic
titration series using affinity biosensors",
ANALYTICAL BIOCHEMISTRY, ACADEMIC
PRESS INC, NEW YORK, vol. 349, no. 1, 1
February 2006 (2006-02-01), pages 136-147,
XP024941980, ISSN: 0003-2697, DOI:
10.1016/J.AB.2005.09.034 [retrieved on
2006-02-01]**
• **Ge Healthcare Life Sciences: "Biacore T200", , 1
June 2010 (2010-06-01), pages 1-8, XP055164885,
Retrieved from the Internet:
URL:http://www.gelifesciences.co.jp/catalo
g/pdf/28979415ab.pdf [retrieved on 2015-01-26]**
• **SIGMUNDSSON K. ET AL.: 'Determination of
active concentration and association and
dissociation rate constants of interacting
biomolecules: an analytical solution to the theory
for kinetic and mass transport limitations in
biosensor technology ant its experimental
verifications' BIOCHEMISTRY vol. 41, 2002,
pages 8263 - 8276, XP008155383**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• KARLSSON R. ET AL.: 'Analysis of active antibody concentrations. Separation of affinity and concentration parameters' JOURNAL OF IMMUNOLOGICAL METHODS vol. 166, 1993, pages 75 - 84, XP023656786

• KAZEMIER B. ET AL.: 'Determination of active single chain antibody concentration in crude periplasmic fractions' JOURNAL OF IMMUNOLOGICAL METHODS vol. 194, no. 2, 1996, pages 201 - 209, XP004021244

## Description

### Field of the invention

The present invention relates to the determination of the concentration of a bioanalyte, such as a protein, and more particularly to the determination of the active concentration of the bioanalyte.

### Background of the invention

There are numerous ways to determine the concentration of proteins and other biomolecules, the majority of the methods involving comparison of the sample to a standard preparation. In many cases, however, no standard is available or the activity of the standard is uncertain.

Many times it is also of importance to know the active concentration of bioanalytes rather than the total concentration which may include functionally inactive molecules. This is, for instance, the case in the development and production of biotherapeutics. However, many established methods for measurement of protein concentration do not distinguish between active and inactive molecules.

Thus, whereas the total concentration of e.g. a protein is typically measured by UV or NIR absorption spectrometry which do not distinguish between active and inactive molecules, the active concentration of a biomolecule may conveniently be measured by biosensor technology, wherein a sample containing the biomolecule is contacted with a sensor surface with a specific ligand immobilized thereon, and the association/dissociation process at the surface is monitored. In this case it is the choice of ligand that defines the activity being measured.

Conventionally, active concentration is measured using a calibration curve. In a development of the determination of active concentration using biosensor technology, however, the analyte concentration can be determined without reference to a calibration standard, using the relationship between the diffusion properties of the analyte and the analyte concentration. Thus, if the diffusion coefficient of the analyte is known, the analyte concentration can be calculated. This type of concentration measurement, which can be useful when no satisfactory calibrant is available for an analyte under study, is usually referred to as Calibration-Free Concentration Analysis (CFCA), and relies upon measurement of analyte binding at varying flow rates under conditions where the observed rate of binding is partially or completely limited by transport of analyte molecules to the sensor surface, i.e. partially or completely controlled by diffusion.

Using surface plasmon resonance detection, CFCA was first described by Karlsson, R., et al. (1993), J. Immunol. Methods 166(1):75-8, and further by Sigmundsson, K., et al. (2002) Biochemistry 41(26):8263-76. This methodology has been implemented in the commercial Biacore® systems (marketed by GE Healthcare, Uppsala, Sweden).

In the Biacore® instruments, samples are injected on a micro-flow system and transported by laminar flow to the sensor surface. Molecules reach the sensor surface from bulk solution by a diffusion controlled transport process. In addition to the concentration of analyte molecules, factors influencing the transport rate include the diffusion coefficient, flow cell dimensions, and flow rate. The balance between the transport rate and the binding rate determines whether the observed binding will be transport limited or reaction limited. For successful CFCA, the observed binding rate must, as mentioned above, be at least partially limited by transport.

It is an object of the present invention to provide an improvement of the above described method for calibration-free concentration analysis under partial or complete transport limitation.

### Summary of the invention

The above mentioned object as well as other objects and advantages are obtained by a method for on determination of active concentration based on the method for CFCA as outlined above but where measurements are made at multiple sample dilutions and several (or all) dilutions are included in global fit, the same fitting criteria being applied to several dilutions. This makes the analysis more robust and extends the dynamic range.

In one aspect, the present invention therefore provides a method of determining active concentration of an analyte in a liquid sample, comprising the steps of:

(a) contacting a laminar flow of the sample with a solid phase surface or surface area supporting a ligand capable of specifically binding the analyte at a plurality of different flow rates and under partially or completely mass transport limited conditions;
(b) determining the initial binding rate dR/dt of analyte to the ligand at the ligand-supporting surface or surface area, and
(c) fitting the initial binding rate data obtained in step (b) to a kinetic interaction model that includes a term for mass transport to obtain the active analyte concentration,
wherein steps (a) and (b) are performed at a plurality of different dilutions of the liquid sample, and

wherein in step (c) at least several of the plurality of dilutions of the liquid sample are in included in a global fit of initial binding rate data to the kinetic interaction model and wherein the different flow rates in step (a) are obtained by varying the flow rate during a single contacting cycle, and further comprising contacting a laminar flow of the liquid sample with a plurality of solid phase surfaces or surface areas, each surface or surface area having a different ligand density, determining from the initial binding rates the initial binding rate corresponding to transport-limited interaction at the surfaces or surface areas, and from that binding rate determining the active analyte concentration.

In a preferred embodiment, the method is performed at two substantially different flow rates.

In still another preferred embodiment, at least three, and preferably at least five different sample dilutions are used.

Other preferred embodiments are set forth in the dependent claims.

The method of the invention may conveniently be implemented by software run on an electrical data processing device, such as a computer. Such software may be provided to the computer on any suitable computer-readable medium, including a record medium, a read-only memory, or an electrical or optical signal which may be conveyed via electrical or optical cable or by radio or other means.

Another aspect of the invention therefore relates to a computer program product comprising instructions for causing a computer to perform the method steps of the mentioned method aspect above.

A more complete understanding of the present invention, as well as further features and advantages thereof, will be obtained by reference to the following detailed description and the accompanying drawings.

**Brief description of the drawings**

Figure 1 is a plot of fitted binding versus time data for two different flow rates in conventional CFCA.

Figure 2 is a plot of globally fitted binding versus time data at several concentrations analysed at the same time.

**Detailed description of the invention**

Unless defined otherwise, all technical and scientific terms used herein have the same meaning as commonly understood by a person skilled in the art related to this invention. Also, the singular forms "a", "an", and "the" are meant to include plural reference unless it is stated otherwise.

As mentioned above, the present invention relates to the determination of the active concentration of an analyte, typically a bioanalyte, in a fluid sample. In brief, the method comprises performing calibration-free concentration analysis under at least partial transport limitation on several dilutions of a liquid sample containing the analyte whose concentration is to be determined, and including several or all dilutions in a global fit of concentration data, the same fitting criteria being applied to several dilutions.

Preferably, an interaction analysis sensor is used in the active concentration determination, typically a biosensor.

Before describing the present invention in more detail, the concept of biosensors and the detection of interactions kinetics will be briefly described.

A biosensor is typically based on label-free techniques, detecting a change in a property of a sensor surface, such as mass, refractive index or thickness of the immobilized layer. Typical biosensors for the purposes of the present invention are based on mass detection at the sensor surface and include especially optical methods and piezoelectric or acoustic wave methods. Representative sensors based on optical detection methods include those that detect mass surface concentration, such as sensors based on reflection-optical methods, including e.g. evanescent wave-based sensors including surface plasmon resonance (SPR) sensors; frustrated total reflection (FTR) sensors, and waveguide sensors, including e.g. reflective interference spectroscopy (RIfS) sensors. Piezoelectric and acoustic wave sensors include surface acoustic wave (SAW) and quartz crystal microbalance (QCM) sensors.

Biosensor systems based on SPR and other detection techniques are commercially available. Exemplary such SPR-biosensors include the flow-through cell-based Biacore® systems (GE Healthcare, Uppsala, Sweden) and Prote-On™ XPR system (Bio-Rad Laboratories, Hercules, CA, USA) which use surface plasmon resonance for detecting interactions between molecules in a sample and molecular structures immobilized on a sensing surface. As sample is passed over the sensor surface, the progress of binding directly reflects the rate at which the interaction occurs. Injection of sample is usually followed by a buffer flow during which the detector response reflects the rate of dissociation of the complex on the surface. A typical output from the system is a graph or curve describing the progress of the molecular interaction with time, including an association phase part and a dissociation phase part. This binding curve, which is usually displayed on a computer screen, is often referred to as a "sensorgram".

With the Biacore® systems it is thus possible to determine in real time without the use of labeling, and often without purification of the substances involved, not only the presence and concentration of a particular molecule, or analyte, in a sample, but also additional interaction parameters, including kinetic rate constants for association (binding) and dissociation in the molecular interaction as well as the affinity for the surface interaction.

In the following, the present invention will to a large extent be described, for illustration only and no limitation, with reference to SPR sensors of the Biacore® system type.

As mentioned above, the Biacore® systems, as well as analogous sensor systems, measure the active analyte concentration as distinct from the total concentration of the analyte, the choice of ligand on the sensor surface defining the kind of activity being measured.

When an analyte is injected into a laminar flow system, e.g. of the Biacore® system type, in such a way that the analyte contacts a sensor surface, it will give rise to a binding event. The rate of the analyte/ligand interaction will be determined by the interaction kinetics and by the transport efficiency of the flow system.

For a biochemical interaction the rate at which an interaction proceeds is given by the difference between the forward (association) and the reverse (dissociation) processes. For a reversible 1:1 interaction between an analyte A and a surface-bound (immobilised) capturing molecule or ligand B which is not diffusion or mass transfer limited

$$A + B \underset{k_d}{\overset{k_a}{\rightleftharpoons}} AB \qquad (1)$$

where $k_a$ and $k_d$ are the rate constants for the association and dissociation, respectively.

The association rate is given by $k_a[A][B]$ and the dissociation rate is given by $k_d[AB]$. The net rate of binding (i.e. the change in surface concentration of formed complex B) is therefore

$$\frac{d[AB]}{dt} = k_a[A][B] - k_d[AB] \qquad (2)$$

After a time t, the concentration of unbound ligand B at the surface is $[B_T] - [AB]$, where $[B_T]$ is the total, or maximum, concentration of ligand B. Insertion into equation (2) above gives

$$\frac{d[AB]}{dt} = k_a[A]\{[B_T] - [AB]\} - k_d[AB] \qquad (3)$$

In terms of detector response units (in Biacore® systems formation of complex is observed as an increase in response, measured in resonance units (RU)), this can be expressed as

$$\frac{dR}{dt} = k_a C(R_{max} - R) - k_d R \qquad (4)$$

were R is the response in response units, C is the concentration of analyte in the sample, and $R_{max}$ is the response obtained if analyte (A) had bound to all ligand (B) on the surface, i.e. the maximum analyte binding capacity.

The kinetic rate constants $k_a$ and $k_d$ are typically calculated by fitting response data for, preferably, a number of different concentrations of analyte and, preferably, also at least one other ligand density at the sensor surface to equation (4) above, or to the integrated form thereof:

$$R = \frac{k_a C R_{max}}{k_a C + k_d}(1 - e^{-(k_a C + k_d)t} \qquad (5)$$

Software for the analysis of kinetic and affinity data is commercially available. Thus, for example, evaluation of kinetic and affinity data produced by the Biacore® instruments is usually performed with the dedicated BIAevaluation software (supplied by GE Healthcare, Uppsala, Sweden) using numerical integration to calculate the differential rate equations and non-linear regression to fit the kinetic and affinity parameters by finding values for the variables that give the closest fit, reducing the sum of squared residuals to a minimum. The "residuals" are the difference between the calculated and the experimental curve at each point, squared residuals being used to weight equally deviations above and below the experimental curve. The sum of squared residual is expressed by the equation:

$$S = \sum_{l}^{n} (r_f - r_x)^2 \qquad (6)$$

where S is the sum of squared residuals, $r_f$ is the fitted value at a given point, and $r_x$ is the experimental value at the same point. For example, for the molecular interaction described above, such software-assisted data analysis is performed by, after subtracting background noises, making an attempt to fit the above-mentioned simple 1 : 1 binding model as expressed by equations (4) or (5) above to the measurement data.

Usually the binding model is fitted simultaneously to multiple binding curves obtained with different analyte concentrations C (and/or with different levels of surface derivatization $R_{max}$). This is referred to as "global fitting", and based on the sensorgram data such global fitting establishes whether a single global $k_a$ or $k_d$ will provide a good fit to all the data.

The above is, however, only valid for a reaction which is not diffusion or mass transfer limited.

Thus, for analyte to bind to the sensor surface, the molecule must be transported from the bulk solution to the sensor surface, which is a diffusion limited process. Under conditions of laminar flow, which apply in the Biacore® and analogous biosensor systems, the transport rate is proportional to the concentration of analyte in the bulk solution.

In a given analysis situation, the observed rate of binding at any time will be determined by the relative magnitudes of the net biochemical interaction rate and the rate of mass transport. If interaction is much faster than transport, the observed binding will be limited entirely by the transport processes. This is also the case when the analyte does not diffuse fast enough from the surface during dissociation, leading to re-binding. Conversely, if transport is fast and interaction is slow, the observed binding will represent the interaction kinetics alone. When the rates of the two processes are of similar orders of magnitude, the binding will be determined by a combination of the two rate characteristics.

The overall interaction process can be represented by the scheme

$$\mathrm{A_{bulk}} \leftrightarrow \mathrm{A_{surface}} + \mathrm{B} \leftrightarrow \mathrm{AB} \qquad (7)$$

The rate of mass transport from bulk solution to the surface is given by

$$\frac{d[A_{surface}]}{dt} = k_m [A_{bulk}] \qquad (8)$$

where $A_{surface}$ is the analyte concentration at the sensor surface, $A_{bulk}$ is the analyte concentration in the bulk solution and $k_m$ is the mass transport coefficient.

The differential equation describing the binding interaction will therefore include a term for mass transfer of analyte to the surface corresponding to equation (8) above. For a flow cell, a"two- compartment" model consisting of a set of coupled ordinary differential equations and described in, for example, Myszka, D. G. et al. (1998) Biophys. J. 75,583-594 is considered to give a reasonable description of the binding kinetics when the data are influenced by mass transport. In this model, the flow cell is assumed to be divided into two compartments, one in which the concentration of analyte is constant, and a second near the sensor surface where the analyte concentration depends on the mass transport rate, the surface density of ligand, and the reaction rate constants.

For the interaction exemplified above of a monovalent analyte A reacting with an immobilised monovalent ligand B, this model may be represented by the following two differential equations replacing equation (3) above

$$\frac{d[A_{surface}]}{dt} = (-k_a A_{surface}(B_T - AB) + k_d AB + k_m (A_{bulk} - A) \qquad (9)$$

$$\frac{dAB}{dt} = k_a A_{surface}(B_T - AB) - k_d AB \qquad (10)$$

where $k_m$ is the mass transport coefficient (describing diffusive movement of analyte between the compartments), $B_T$ is the total ligand concentration, Surface is the concentration of free analyte at the sensor surface, $A_{bulk}$ is the injection (i. e. initial) analyte concentration, AB is the concentration of complex AB (= surface density of bound analyte), and $k_a$ and $k_d$ are the association and dissociation rate constants, respectively.

As will be described in more detail below, the mass transport coefficient $k_m$ can be calculated, and fitting of response data to equations (9) and (10) will give the kinetic rate constants $k_a$ and $k_d$.

The kinetic characterizations outlined above have traditionally been performed using either the well-established method where each sample concentration is run in a separate cycle, and analyte is removed by regeneration of the surface between each cycle. In a more recently developed approach, however, referred to as "single cycle analysis", the analyte is injected with increasing (or otherwise varied) concentrations in a single cycle, the surface not being regenerated between injections. For a more detailed description of such single cycle analysis it may be referred to Karlsson, R., et al. (2006) Anal. Biochem. 349:136-147.

For the above-mentioned mass transport coefficient $k_m$, the following equation applies

$$k_m = 0.98 \times \left(\frac{D}{h}\right)^{2/3} \left(\frac{F}{0.3 \times w \times l}\right)^{1/3} \qquad (11)$$

where D is the diffusion coefficient ($m^2/s$) of the analyte, F is the volumetric flow rate of liquid through the flow cell ($m^3/s$), and h, w and 1 are the flow cell dimensions (height, width, length (m)).

The diffusion coefficient D is a function of the size and shape of the molecule and the frictional resistance offered by the viscosity of the solvent in question. For spherical molecules, the diffusion coefficient is inversely proportional to the radius and thus proportional to the cube root of the molecular weight. For very large solute molecules, such as proteins, however, the diffusion coefficient is relatively insensitive to the molecular weight.

If there is no literature value for the diffusion coefficient, it may determined experimentally, e.g. by analytical ultracentrifugation or light scattering.

Alternatively, the diffusion coefficient may be estimated from the molecular weight and the shape factor, or frictional rate, according to the equation

$$D = 342.3 \times \frac{1}{M^{1/3} \times f \times \eta_{rel}} \times 10^{-11} \qquad (12)$$

where D is the diffusion coefficient ($m^2/s$), M is the molecular weight (daltons), f is the frictional ratio, and $\eta_{rel}$ is the viscosity of the solvent relative to water at 20° C.

From equations (9) and (10), the mass transport coefficient $k_m$ can thus be calculated.

For Biacore® systems, a Biacore-specific mass transfer constant kt may be obtained by adjusting for the molecular weight of the analyte and conversion from measured response (in RU) to concentration units:

$$k_t = k_m \times M \times 10^9 \qquad (13)$$

where the conversion constant $10^9$ is approximate and only valid for protein analytes and a specific sensor surface, Sensor Chip CM5 (GE Healthcare, Uppsala, Sweden). For other analytes/sensor surfaces a calibration of the system with a RU conversion factor is required (1 RU equals x $ng/mm^2$).

For evaluation of calibration-free concentration measurements, using the relationship between the diffusion properties of the analyte together with analysisi of the binding rate under partially diffusion-controlled conditions, the mass transport coefficient is calculated from the diffusion coefficient, and then converted to the mass transport constant kt which is used in fitting the experimental data to the diffusion-controlled interaction model whereby the analyte concentration of the sample can be obtained.

More particularly, in calibration-free concentration assays (CFCA), a liquid sample containing the analyte, e.g. a protein, is run at several flow rates against an immobilized ligand, the initial binding rate (dR/dt) of each run being determined, in the case of a Biacore® system using SPR detection technology. The analyte concentration C is then evaluated, typically by dedicated software, by fitting the binding data to a model with a global variable for the analyte concentration. That is, setting the analyte concentration as a parameter to fit and $k_m$ as a known constant together with the molecular weight Mw, so that the model is constrained to find a single concentration value that best fits all binding curves simultaneously.

Usually, it is sufficient to use only two flow rates, provided that they are widely separated, preferably as widely separated as the system used will allow.

Thus, the current implementation of the technology in Biacore® systems involves two binding experiments where analyte binds to immobilized ligand; one experiment at a low flow rate (often 5 or 10 μl/min) and one experiment at a high flow rate (often 100 μl/min). In the analysis, responses are checked for transport (diffusion) limited behavior and the response is fitted to a kinetic model where the transport coefficient is constant and the concentration of the analyte is fitted. (For details it is referred to 28-9768-788A Biacore T200 Software Handbook [GE Healthcare, Uppsala, Sweden],

Generally, binding rates should be measured shortly after start of the sample injection, since the rates approach zero as the binding approaches a steady state.

Calibration-free assays are usually set up a direct binding format in order to obtain the high binding capacities that are usually required.

### *The invention*

In typical CFCA experiments, several dilutions of the sample are analysed and evaluated separately. According to the present invention, a more robust analysis and one which has an extended dynamic range is obtained by including several (e.g. at least three) or all dilutions in a global fit of concentration data, so that the same fitting criteria are applied to several dilutions of the sample. This will also facilitate sensitivity analysis of the results as it becomes possible to determine whether other concentration values can be used to obtain identical or similar quality of global analysis.

As an illustration of how the method of the invention may be performed with Biacore® system, the scripting language used in the dedicated Biaevaluation software may, for example, be as follows:

The total response comes from complex formation and from refractive index change

$$AB+\$1*RI.$$

Short expressions define:

$1 - When injection takes place $1 expression is equal to one during injection between ton1 and toff1 otherwise zero.

$$\$1=(sign(t\text{-}ton1)\text{-}sign(t\text{-}toff1))/2;$$

S2 describes the transport of analyte to the sensor surface. kt corresponds to the transport coefficient at one μl/min. F1 is the flow rate. Conc is the stock concentration of the sample. Dil is the dilution factor that gives the concentration in the actual sample. A is the conc of analyte at the sensor surface.

$$\$2=F1^{\wedge}(1/3)*kt*(Conc/Dil*\$1\text{-}A);$$

$3 is just the rate equation.

$$\$3=ka*A*B\text{-}kd*AB;$$

A describes how the the analyte concentration varies over time. The starting value is zero.

$$A=\$2\text{-}\$3|0;$$

B describes how the ligand concentration changes over time. The starting value is Rmax.

$$B = -\$3 \,|\, Rmax;$$

AB is how the complex is formed.

$$AB = \$3 \,|\, 0;$$

kt, F1, Dil, ton1, toff1 are constants;
ka, kd, Rmax are fitted globally (Rmax can be locally/globally fitted to subgroups when several ligand densities are used);
C is fitted globally.

With "global data" it becomes possible to perform sensitivity analysis of the fit. This can be similar to U value analysis in kinetic analysis.

This may, for instance be done by taking the Conc value determined, say, 100 nM, then refitting the data with changed Conc that is input as a constant, for instance 80 nM, and refitting the data using the same floating parameters as before. A computer routine may display overlay plots, residuals or chi 2 and highlight when a significant change has taken place.

In a similar way CFCA in single cycle mode is:

$$AB + offset*\$2;$$

$$\$1 = (sign(t\text{-}ton1)\text{-}sign(t\text{-}tonoff))/2;$$

$$\$2 = (sign(t\text{-}tonoff)\text{-}sign(t\text{-}toff2))/2;$$

$$\$3 = kt1*\$1*(Conc\text{-}A) + kt2*\$2*(Conc\text{-}A);$$

$$\$4 = ka*A*B\text{-}kd*AB;$$

$$A = \$3\text{-}\$4 \,|\, 0;$$

$$B = -\$4 \,|\, Rmax;$$

$$AB = \$4 \,|\, 0$$

While in the method of the invention sample injections may be made for each different flow rate, the need to inject samples in two or more cycles may be overcome by the "single cycle" approach described above, where the flow rate varies during one sample injection. For instance, the sample may be injected at 10 $\mu$l/min, and after 5-20 seconds the flow rate is increased to 100 $\mu$l/min. In the data analysis, this is handled by input of actual flow rates in the evaluation algorithms used.

The method of the invention may, optionally, be combined with a newly developed calibration-free concentration analysis method where sensor surfaces or surface areas with varying ligand density (i.e. an array of different ligand densities) are used and a fitting algorithm for determining the maximum initial binding rate and thereby the active concentration is provided.

This method, which is described in our co-pending application "Method of determining active concentration" filed on even date with the present application, is based on using the following equation which applies for a reaction that is totally limited by the transport efficiency of the system:

$$\frac{dR}{dt} = k_m C \qquad\qquad (14)$$

where R is the detector response, $k_m$ is the mass transport coefficient, and C is the analyte concentration of the sample. That is, the response increase dR/dt, or binding rate, at the sensor surface is proportional to the mass transport coefficient and the active concentration of the analyte. From equation (14) it is seen that if the (initial) binding rate dR/dt at total transport limitation and $k_m$ are known, the analyte concentration C may be calculated by dividing dR/dt by $k_m$.

More specifically, in that method, initial binding rates are measured at a number of (preferably at least four) different ligand density levels, i.e. immobilization levels. For each immobilization level, the response is recorded using at least one fixed flow rate. The initial binding rate is plotted versus the immobilization level, or maximal binding capacity $R_{max}$, and the binding rate where dR/dt has reached its maximum is determined by extrapolation of the data, typically using an algorithm capable thereof. This maximum binding rate corresponds to the binding rate at mass transport limitation, meaning that equation (14) above applies, and the active concentration C can therefore be calculated by dividing dR/dt by $k_m$.

Such combined analysis will thus take into account both analysis under partial transport limitation (according to the method of the present invention) and extrapolation to complete transport limitation (as outlined above) and therefore has the potential to become more robust.

The contacting of the sample with surfaces or surface areas with different ligand densities may be performed in an analytical instrument having a single sensor surface by sequential sample injections and varying the ligand density between injections. Preferably, however, the method is performed with a multi-flow channel instrument, such as the Biacore™ T100, T200 or 4000 (GE Healthcare, Uppsala, Sweden) or ProteOn™ XPR system (Bio-Rad Laboratories, Hercules, CA, USA), preferably by parallel sample injections.

In an advantageous method of determining active concentration in a calibration-free format, (initial) binding rate data are collected at different immobilization levels and at different flow rates at partial or complete transport limitation for a number of different sample dilutions. One then has the choice to evaluate the obtained binding rate data according to several alternatives, i.e. either by (i) analysis under partial transport limitation with global fitting of data for several dilutions of a sample; or (ii) extrapolation to complete transport limitation; or (iii) a combination of (i) and (ii).

The invention will now be illustrated further by means of the following non-limiting examples.

## EXAMPLES

### Example 1

### *Global fit of CFCA data*

A procedure for determining active concentration by the method of the present invention using, for example, a modified Biacore® T200 system may be performed as follows.

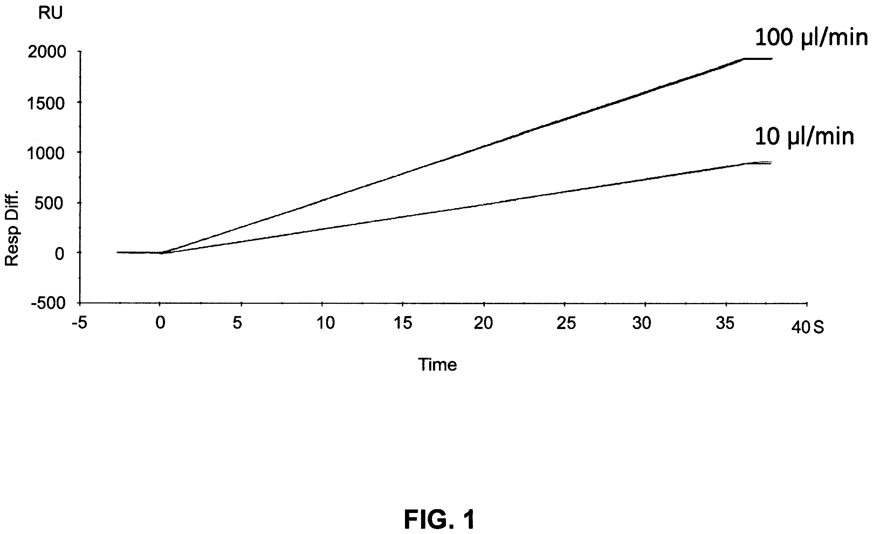

The experiments described here demonstrate antibody binding to an immobilized protein A derivative capable of binding antibody. The conventional CFCA analysis is illustrated in Fig. 1.

The analyte was injected in separate cycles at varying flow rates. In this case the analyte was diluted 400 times relative to its stock concentration. CFCA analysis gives the local antibody concentration as 19.1 nM and thus the stock solution is 7.6 μM.

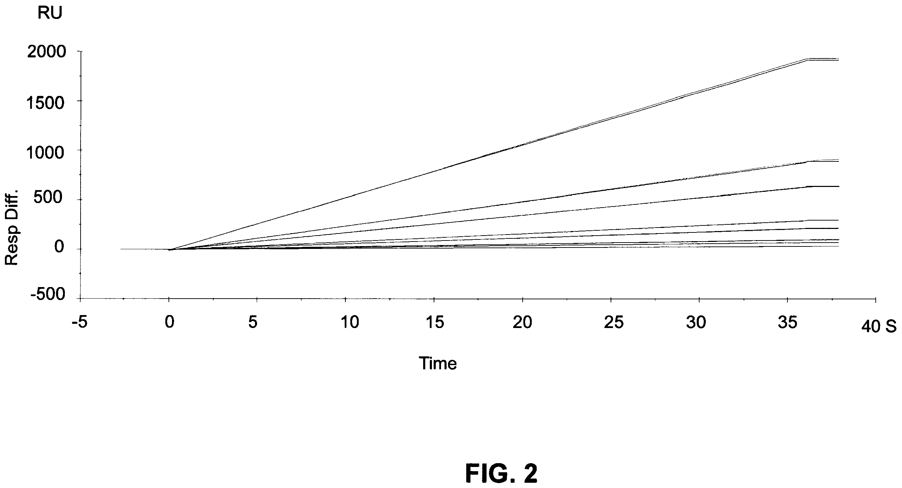

In many cases, however, it is useful to test several dilutions of the sample and analysis becomes tedious. By turning to a global fit of the data several concentrations are analysed at the same time, as shown in Fig. 2.

This graph illustrates the global fit of antibody dilutions 1:400, 1:1200, 1:3600 and 1:10800. Each dilution is injected at two flow rates and the globally determined concentration for the stock solution is 7.6 μM. This immediately demonstrates that the concentration analysis is not dilution dependent and simplifies the analysis.

The present invention is not limited to the above-described preferred embodiments. Various alternatives, modifications and equivalents may be used. Therefore, the above embodiments should not be taken as limiting the scope of the invention, which is defined by the appending claims.

## Claims

1. A method of determining active concentration of an analyte in a liquid sample, comprising the steps of:

(a) contacting a laminar flow of the sample with a solid phase surface or surface area supporting a ligand capable of specifically binding the analyte at at least two different flow rates and under partially or completely mass transport limited conditions;

(b) determining the initial binding rate dR/dt of analyte to the ligand at the ligand-supporting surface or surface area, and

(c) fitting the initial binding rate data obtained in step (b) to a kinetic interaction model that includes a term for mass transport to obtain the active analyte concentration,

wherein steps (a) and (b) are performed at a plurality of different dilutions of the liquid sample, and

wherein in step (c) at least several of the plurality of dilutions of the liquid sample are in included in a global fit of initial binding rate data to the kinetic interaction model and wherein the different flow rates in step (a) are obtained by varying the flow rate during a single contacting cycle, and further comprising contacting a laminar flow of the liquid sample with a plurality of solid phase surfaces or surface areas, each surface or surface area having a different ligand density, determining from the initial binding rates the initial binding rate corresponding to transport-limited interaction at the surfaces or surface areas, and from that binding rate determining the active analyte concentration

2. The method according to claim 1, wherein the method is performed at two substantially different flow rates.

3. The method according to claim 1 or 2, wherein at least three, and preferably at least five different sample dilutions are used.

4. The method according to any one of claims 1 to 3, wherein an interaction analysis sensor is used, preferably a biosensor.

5. The method according to claim 4, wherein the interaction analysis sensor is based on mass-sensing, preferably evanescent wave sensing, especially surface plasmon resonance (SPR).

6. The method according to any one of claims 1 to 5, which is computer-implemented.

7. A computer program product comprising instructions for causing a computer to perform the method steps of any one of claims 1 to 5, when said program is run on a data-processing system.


**Patentansprüche**

1. Verfahren zur Bestimmung einer aktiven Konzentration eines Analyten in einer flüssigen Probe, umfassend die Schritte:

(a) Kontaktieren eines Laminarflusses der Probe mit einer Festphasenfläche oder einem Festphasenflächenbereich, die bzw. der einen Liganden trägt, welcher in der Lage ist, den Analyten bei mindestens zwei verschiedenen Flussraten und unter teilweise oder vollständig Massetransport-begrenzten Bedingungen spezifisch zu binden;

(b) Bestimmen der anfänglichen Bindungsrate dR/dt des Analyten an den Liganden an der ligandentragenden Fläche bzw. dem ligandentragenden Flächenbereich und

(c) Anpassen der in Schritt (b) erhaltenen Daten der anfänglichen Bindungsrate an ein kinetisches Interaktionsmodell, das einen Term für Massetransport aufweist, um die aktive Analytkonzentration zu erhalten,

wobei die Schritte (a) und (b) bei einer Mehrzahl von verschiedenen Verdünnungen der flüssigen Probe durchgeführt werden und

wobei in Schritt (c) mindestens mehrere der Mehrzahl an Verdünnungen der flüssigen Probe in eine Gesamtanpassung von Daten der anfänglichen Bindungsrate an das kinetische Interaktionsmodell aufgenommen werden und wobei die verschiedenen Flussraten in Schritt (a) erhalten werden, indem die Flussrate während eines einzelnen Kontaktierungszyklus variiert wird, und weiter umfassend das Kontaktieren eines Laminarflusses der flüssigen Probe mit einer Mehrzahl an Festphasenflächen bzw. - flächenbereichen, wobei jeder Fläche bzw. jeder Flächenbereich eine andere Ligandendichte hat, das Bestimmen der anfänglichen Bindungsrate, die einer transportbegrenzten Interaktion an den Flächen bzw. Flächenbereichen entspricht, aus den anfänglichen Bindungsraten und davon ausgehend der Bindungsrate, welche die aktive Analytkonzentration bestimmt.

2. Verfahren nach Anspruch 1, wobei das Verfahren bei zwei im Wesentlichen verschiedenen Flussraten durchgeführt wird.

3. Verfahren nach Anspruch 1 oder 2, wobei mindestens drei und vorzugsweise mindestens fünf verschiedene Probenverdünnungen verwendet werden.

4. Verfahren nach einem der Ansprüche 1 bis 3, wobei ein Interaktionsanalysesensor, vorzugsweise ein Biosensor, verwendet wird.

5. Verfahren nach Anspruch 4, wobei der Interaktionsanalysesensor auf einer Masseerfassung, vorzugsweise auf der Erfassung evaneszenter Wellen, insbesondere auf Oberflächenplasmonresonanz (SPR), beruht.

6. Verfahren nach einem der Ansprüche 1 bis 5, das rechnerimplementiert ist.

7. Rechnerprogrammprodukt, umfassend Anweisungen zum Veranlassen eines Rechners, die Verfahrensschritte nach einem der Ansprüche 1 bis 5 durchzuführen, wobei das Programm auf einem datenverarbeitenden System läuft.


**Revendications**

1. Procédé de détermination de la concentration active d'un analyte dans un échantillon liquide, comprenant les étapes de :

   (a) mettre en contact un flux laminaire de l'échantillon avec une surface ou une superficie de phase solide supportant un ligand capable de se lier spécifiquement à l'analyte à au moins deux débits différents et dans des conditions partiellement ou complètement limitées de transport de masse ;
   (b) déterminer le taux de liaison initial dR/dt de l'analyte au ligand au niveau de la surface ou superficie supportant le ligand, et
   (c) ajuster les données du taux de liaison initial obtenues dans l'étape (b) à un modèle d'interaction cinétique qui inclut un terme pour le transport de masse pour obtenir la concentration d'analyte actif,

   dans lequel les étapes (a) et (b) sont effectuées à une pluralité de dilutions différentes de l'échantillon liquide, et dans lequel dans l'étape (c) au moins plusieurs de la pluralité de dilutions de l'échantillon liquide sont incluses dans un ajustement global des données du taux de liaison initial au modèle d'interaction cinétique et dans lequel les débits différents dans l'étape (a) sont obtenus en faisant varier le débit pendant un cycle de contact unique, et comprenant en outre mettre en contact un flux laminaire de l'échantillon liquide avec une pluralité de surfaces ou superficies de phase solide, chaque surface ou superficie ayant une densité de ligand différente, déterminer à partir des taux de liaison initiaux le taux de liaison initial correspondant à l'interaction limitée au transport au niveau des surfaces ou superficies, et à partir de ce taux de liaison déterminer la concentration d'analyte active.

2. Procédé selon la revendication 1, dans lequel le procédé est effectué à deux débits sensiblement différents.

3. Procédé selon la revendication 1 ou 2, dans lequel au moins trois, et de préférence au moins cinq dilutions d'échantillon différentes sont utilisées.

4. Procédé selon l'une quelconque des revendications 1 à 3, dans lequel un capteur d'analyse d'interaction est utilisé, de préférence un biocapteur.

5. Procédé selon la revendication 4, dans lequel le capteur d'analyse d'interaction est basé sur la détection de masse, de préférence la détection de vague évanescente, en particulier la résonance plasmonique de surface (SPR).

6. Procédé selon l'une quelconque des revendications 1 à 15, qui est mis en oeuvre par ordinateur.

7. Produit programme d'ordinateur comprenant des instructions pour amener un ordinateur à effectuer le étapes du procédé selon l'une quelconque des revendications 1 à 5, lorsque ledit programme est exécuté sur un système informatique.

FIG. 1

**FIG. 2**

**EP 2 726 876 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KARLSSON, R. et al.** *J. Immunol. Methods,* 1993, vol. 166 (1), 75-8
- **SIGMUNDSSON, K. et al.** *Biochemistry,* 2002, vol. 41 (26), 8263-76
- **MYSZKA, D. G. et al.** *Biophys. J.,* 1998, vol. 75, 583-594
- **KARLSSON, R. et al.** *Anal. Biochem.,* 2006, vol. 349, 136-147